# EUROPEAN PATENT APPLICATION

(11) **EP 2 412 305 A1**
(43) Date of publication of application: **01.02.2012**
(21) Application number: 10290429.9
(22) Date of filing: 30.07.2010
(51) Int. Cl.: A61B 3/10, A61B 3/16, A61B 5/00

(54) **Integrated flexible passive sensor in a soft contact lens for IOP monitoring**

(71) Applicant: Ophtimalia, 14460 Colombelles (FR)
(72) Inventor: Auvray, Philippe, 14000 Caen (FR); Lissorgues, Gaelle, 94170 Le Perreux sur Marne (FR); Rousseau, Lionel, 94170 Le Perreux sur Marne (FR)
(74) Representative: Grünecker, Kinkeldey, Stockmair & Schwanhäusser

(57) **Abstract**

The invention relates to a sensing means for a contact lens used in a physiological parameter measuring system comprising one or two contact lenses, wherein to improve the comfort of wear for a patient each contact lens comprises an antenna and a purely passive sensing means. The invention also relates to a corresponding method and a method to fabricate the sensing means and the contact lens.

## Description

The invention relates to a passive sensor means, in particular for measuring a physiological parameter, e.g. the intraocular eye pressure (IOP), that can be incorporated into or onto a contact lens, in particular a soft contact lens. The invention also relates to a fabrication method and a method to measure the physiological parameter.

Intraocular pressure is a physiological parameter used to diagnose glaucoma, which is characterized by an elevated pressure with, in many cases, large variations inside the eye.

Typically, the intraocular pressure is measured using special ophthalmological devices called Tonometers. The measurements can only be carried out in a doctor's practice or at a hospital. As a consequence, this kind of measurement needs the presence of the patient in the practice or at the hospital and the IOP pressure is only measured at a given time during the day. Hence, no information about the evolution of the IOP is obtained during a long period of the day in a patient's normal environment.

There is, therefore, a need to provide an improved measuring and monitoring system that will enable to record possible causes, comprising physical activities, environmental, posture or psychological causes that may have an effect on the variations of the IOP value. At the same time, knowing at any time the value of the physiological parameter under study, it will become possible for patients to not only obtain an improved diagnostic but it will also become possible to follow an adapted medical treatment.

Prior art document WO 03/001991 proposes an intraocular pressure recording system allowing a continuous monitoring over prolonged periods, regardless of a patient's position and activities. The recording system described therein comprises a soft contact lens including an active strain gauge for measuring spherical deformations. The information is transmitted to an external recording system. Due to the use of the active strain gauge on the contact lens, substantial power needs to be provided to the contact lens, thus a rather high radiation level close to the patient's eye can be observed and heat needs to be dissipated. The power consumption leads to a limited autonomy of the system in case of a battery driven use. Furthermore, in case of a wireless system, the energy transmitting device needs to be positioned as close as possible to the user which reduces the wearing comfort for the patient.

There is, therefore, a need to provide an improved sensing means for such an intraocular pressure recording system.

This object is achieved with the sensing means according to claim 1. The inventive sensing means for a contact lens has an inductance comprising first spires on a first main side of a carrier substrate and second spires on the second main surface, opposite the first surface, of the carrier substrate and a capacitance. With a complete passive sensing means, physiological parameters can still be measured based on the shift of the resonance frequency of such a LC circuit by its deformation, e.g. under pressure, wherein by providing spires of the inductance on both sides of the carrier substrate, the resonant frequency of the LC circuit can be shifted towards frequency ranges which are allowed for measurements on the human body. Using purely passive sensing means, the radiation energy dissipation present around the physiological parameter measuring system can be kept low to reduce any risks related to their presence in the vicinity of a patient's eye. The device allows the creation of a medical device that provides a good measurement sensitivity, a improved comfort for the patient and can comply with regulations in term of bio-compatibility and use of frequency bands for the sensor activation. At the same time a thin sensing device can be achieved which is not possible with a design using active elements.

Preferably, the capacitance can be integrally formed with the inductance, wherein the one electrode is on the first main side and the second electrode is on the second main side of the carrier substrate and the dielectric of the capacitance is a part of the carrier substrate. The presence of an integrated capacitance simplifies the fabrication process as the capacitance can be fabricated at the same time as the spires of the inductance.

According to a preferred embodiment, the capacitance can be arranged towards the outside of the spires when looking from the centre of the spires. With this design, once the sensing means attached to a contact lens, the wearing comfort for the patient can be kept high, as the capacitor will not obscure the field of vision of the patient.

Advantageously, the capacitance can be arc-shaped and can extend over less than an entire circle. With this shape the capacitance can be adapted to the shape of a contact lens and by preventing the capacitance to form an entire circle it does not affect the inductance.

Further preferred, the sensing means for a contact lens according can further comprise at least one interdigitated capacitance, in particular connected to the capacitance. Such an additional capacitance typically with interlaced fingers allows for fine tuning of the LC circuit, so that it can be ensured that the resonant frequency is in the correct range. Once the circuit tested, one or more fingers of the interdigitated capacitance can be cut, e.g. by laser ablation to change the properties of the sensing circuit.

Preferably, the interdigitated capacitance can be arc-shaped. Thereby, the desired advantages of the interdigitated capacitance can be kept while at the same time the shape can be adapted to a contact lens, in particular when the interdigitated capacitance is arranged towards the outside of the spires.

Advantageously, the two connecting parts of the spires can be both either arranged towards the inside of the spires or the outside of the spires. In this case the connection to an additional capacitance is simplified. In this context inside or outside relates to the arrangement when looking from the centre of the spires. The arrangement towards the outside is more preferred as at a given arrangement of the inductance, the wearing comfort is improved as the capacitance is kept outside the patient's field of vision.

Preferably, the connecting parts are arranged on the same side of the flexible carrier substrate. If instead of an integrated capacitance an additional element is connected to the spires the connection is simplified, in case the connection is either on the top or the bottom of the carrier substrate.

According to a preferred embodiment, the first spires and the second spires can be connected via a plurality of vias over the entire length of the spires. In this case, the resistivity of the inductance can be reduced as the effective thickness is essentially doubled. The vias can be arranged at a fixed distance with respect to each other.

Advantageously, the sensing means can comprise two crossing areas where an essentially semi circularly shaped part of the spires connects with another essentially semi circularly shaped part in the subsequent round of the spires. This simplifies the design to get the connection parts either on the inside or outside.

Further preferred, the connection between two essentially semi circularly shaped parts of the spires can be either achieved on the first or the second main surface side. Advantageously, the connected essentially semi circularly shaped part of the spires can extend from the outside towards the inside and then back from the inside to the outside. Further preferred each spire going inward can cross only one spire going outward and vice versa. In this case, the connections towards the outside can e.g. be connected on the first surface side, whereas the connections towards the inside can be arranged on the second surface side. In this case one spire build up by two essentially semi circular shaped parts connected with each other and wind towards the outside crosses only one spire build up by two essentially semi circular shaped parts connected with each other and wind towards the inside so that the parasitic capacitance can be kept small.

According to a preferred variant, the first spires and the second spires can be connected via one via at an end of the first and the beginning of the second spires wherein the spires are wound in the same direction to form a series of two inductances. This way the inductance value can be enhanced without needing more area.

Preferably, the inductance can comprise one to twenty five spires, in particular with a thickness of 10µm to 40µm per layer and a width of about 70µm to 100µm. With these values the desired resonant frequencies can be obtained without limiting the patients comfort when wearing the contact lens.

Preferably, the carrier substrate can at least be partially removed in areas without spires and/or the capacitor and/or the interdigitated capacitor. This enhances the flexibility of the sensing means so that the sensitivity by deformation of the sensing means can be improved. The invention furthermore relates to a contact lens for a physiological parameter measuring system comprising one or two contact lenses, comprising a sensing means according to one of the claims described above and which achieves the advantages as described above.

The object is also achieved with the physiological parameter measuring system comprising one or two contact lenses, wherein each contact lens is according to one of the claims described above. In contrast to the prior art, the inventive system proposes the use of a passive sensing means so that the energy consumption and, therefore, the energy transfer towards the contact lens, can be greatly reduced compared to the use of an active strain gauge. Preferably, the physiological parameter is the intraocular eye pressure.

Preferably, the passive sensing means can comprise a capacitor or a transducer, in particular a piezoelectric transducer. Using a capacitor, which can be an off the shelf capacitor or fabricated together with the antenna before being embedded into the contact lens, and thus leads to a simplified design representing a LC circuit. A piezoelectric transducer can replace the capacitor and is arranged in parallel with the antenna. The electrical equivalent model shows that the principle of measuring the variation of the resonant frequency is still valid. This solution offers a higher sensitivity, thus less energy is required to power the sensor. Using this kind of sensing means, unwanted effects of radiation and of energy dissipation, which might be harmful to the patient, can be lowered. At the same time, variations of the physiological parameter can be identified based on the observed resonant frequency shift.

Advantageously, the physiological parameter measuring system can further comprise one or two antenna patches external to the one or two contact lenses. Via the antenna patches an inductive coupling can be achieved with the circuitry of the contact lens so that signals provided by the sensing means can be received by the antenna patch via the antenna of the contact lens. As no cable connection is necessary, the patient's comfort during a measurement series of the IOP is improved compared to measuring systems which are cable based.

Advantageously, the antenna patch can comprise a transparent, in particular flexible, support layer and an adhesive layer. This antenna patch can thus be simply attached to a pair of spectacles, so that they can be positioned in front of the contact lens to ensure an inductive coupling between the antenna of the contact lens and the antenna of the patch.

Further preferred, the physiological parameter measuring system can comprise at least one electronic conditioning unit, in particular one electronic conditioning unit per antenna patch, connected to an antenna patch and configured to supply a stimulus signal to the antenna patch and to receive a response signal received in turn by the antenna patch from the antenna of the contact lens. For instance the stimulus signal can be a sine wave with a varying frequency, thus like a swept sine signal.

Advantageously, the conditioning unit can furthermore be configured to analyse the response signal, in particular in the frequency domain and of the determining phase properties of the response signal. Thereby the influence of the coupling between the contact lens and the antenna patch can be reduced compared to a measurement based on amplitude measurements. Preferably, one takes advantage of changes in the resonance properties of the antenna's passive sensing means of the contact lens to determine the value of the physiological parameter.

Further preferred, in the physiological parameter measuring system, at least one of the at least one electronic conditioning units is connected, in particular wirelessly connected, to a data monitoring unit. The evolution of the physiological parameter can be easily monitored continuously. Preferably, the data monitoring unit can in turn comprise an interface for transmitting the monitored data of the IOP to another electronic device, e.g. over the internet at a distant location.

Further preferred, the electronic conditioning unit/s is/are attached to a branch or the frame of spectacles and each one of the one or two antenna patches is adhered to one glass, in particular on the side of the glasses towards a patient's eye, of the spectacles respectively. Thereby, a compact measuring system, which is easy to wear by a patient, is provided. The data monitoring unit, with its data storage means, can be transported, for instance in a pocket, so that, in the presence of the data monitoring unit, an easily wearable monitoring system is provided. The electronic conditioning unit can be clipped or adhered to the spectacles of a patient.

Further preferred, the adhesive layer can be configured such that the antenna patch is detachable from the glass of the spectacles, preferably without leaving a residue after removal. Thus a patient can use his own glasses as part of the measuring system and remove the antenna patch, once the monitoring has been accomplished.

Preferably, the physiological parameter measuring system can further comprise a filtering unit configured to reduce spurious artifacts from the response signals based on the detection of a simultaneous predetermined change property of the response signal received from the two contact lenses. Thereby, the spurious values can be automatically removed out of the set of measured pressures to improve the accuracy of the measurement. In that case, it is first checked if the response signal is 'normal' by testing it against criteria. This operation is done on each eye individually. If some abnormal behaviour is observed, then the system is configured to check if the response is similar on both eyes. In that case, it can be eye blinking or an eye rotation, for example, the system decides to erase the result and to repeat the measurement.

According to a further preferred variation, the filtering unit can also be configured to improve the signal-to.-noise ration by reducing the contributions of electromagnetic disturbances which can also be eliminated by doing this dual-channel measurement, because they are present on both when occurring. Thus using a bi-ocular measurement one can take advantage of differential measurements.

Preferably the filtering can be performed using digital signal processing after an analog to digital conversion and can therefore offer a high flexibility concerning data treatment.

Further preferred, the two electronic conditioning units can be arranged in a master and slave configuration, in a system using two electronic conditioning units. Using this configuration, one can balance the weight of or on the branches of the spectacles. For instance, one conditioner can comprise the batteries, power management, and a few first part of electronic components, whereas the other one comprises the remaining electronic components, to achieve this balance.

According to a preferred embodiment, the contact lens can be a vision correcting contact lens or the spectacles can have vision correcting glasses. This further improves the user-friendliness of the inventive physiological parameter measuring system, in particular in combination with the patient's own spectacles.

Advantageously, the antenna and the purely passive sensing means can be arranged on both sides of a flexible carrier substrate. Thus without having to use a larger surface the sensitivity of the system can be improved.

Preferably, the antenna and the purely passive sensing means can comprise spires on the on the one side of a flexible carrier substrate and spires on the other side of a flexible carrier substrate, wherein the spires are connected via a plurality of vias with each other. This arrangement reduces the resistivity of the circuit and thus improves the quality factor of the LC circuit.

In a variant, the antenna and the purely passive sensing means can comprise spires on the on the one side of a flexible carrier substrate and spires on the other side of a flexible carrier substrate, wherein the spires are connected by a via to form a first and second inductance arranged in series. This arrangement corresponds to a simplified yet functional design.

Preferably, an end region of the spires can be enlarged such as to form a capacitor with a predetermined capacitance to thereby adjust the resonant frequency of the LC circuit.

The invention further relates to a method for fabrication a contact lens as described above and comprising the steps of a) providing a sensing means as described above, b) removing the carrier substrate at least partially in areas between spires and/or the capacitor and/or the interdigitated capacitor, c) plastically deforming the sensing means to obtain a bended shape corresponding to the shape of a contact lens, and d) attaching the bended sensing means to the contact lens. With this method a contact lens with integrated sensing means can be achieved that provides the necessary sensitivity and at the same an enhanced wearing comfort.

The invention also relates to a method for measuring a physiological parameter, in particular the intraocular eye pressure, comprising the steps of: a). transmitting a stimulus signal from an antenna patch, in particular adhered to the glasses or spectacles, to a purely passive sensing means of a contact lens, b). receiving a response signal from the purely passive sensing means via the antenna patch, and c). determining the physiological parameter based on a resonant frequency and/or phase shift. Using purely passive sensing means, the radiation energy dissipation present around the physiological parameter measuring system can be kept low to reduce any risks related to their presence in the vicinity of a patient's eye. The antenna patch adhered to the glasses allows an improved coupling between the antennas.

Advantageously, the steps a) - c) can be carried out with two antenna patches and two corresponding contact lenses and, furthermore, comprise a filtering step to filter out spurious artifacts, in particular eye blinking or eye rotations leading to a change of the viewing direction, based on a simultaneous predetermined change property in the response signal received from the two contact lenses. By carrying out a bi-ocular measurement, an automatic filtering can be realized to improve the accuracy of the device.

The invention will be described in more detail in the following based on advantageous embodiments described in conjunction with the following Figures:
- Figure 1: illustrates a physiological parameter measuring system according to the invention,
- Figure 2a: schematically illustrates the structure of a contact lens used in a first and second embodiment of the invention,
- Figure 2b: illustrates a first embodiment of the antenna and sensing means of the contact lens according to the invention,
- Figure 2c: illustrates a second embodiment of the antenna and sensing means of the contact lens according to the invention,
- Figure 2d: illustrates a third embodiment of the antenna and sensing means of the contact lens according to the invention,
- Figure 2e: illustrates in detail the inter-digitated capacitor of the third embodiment,
- Figure 2f: illustrates the electric equivalent circuit of the third embodiment,
- Figure 2g: illustrates a fourth embodiment of the antenna and sensing means of the contact lens according to the invention,
- Figure 3: illustrates a partial 3-dimensional view of one of the antennas according to the sixth embodiment of the invention,
- Figure 4: illustrates the electrical equivalent circuit,
- Figure 5: illustrates a method according to a seventh embodiment using the contact lenses according to the invention,
- Figure 6: illustrates a resonance measurement using a practical example according to the invention, and
- Figure 7: illustrates a eighth embodiment according to the invention, wherein the physiological parameter measuring system comprises, in addition, a filtering unit, and
- Figures 8a to 8d: illustrate a fabrication method according to a fifth embodiment of the invention for fabrication a contact lens according to one of the first to fourth embodiment

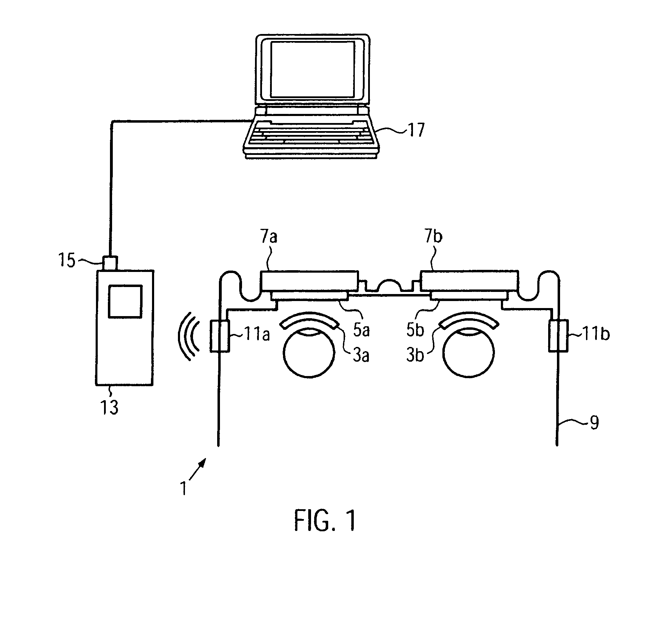

Figure 1 illustrates a physiological parameter measuring system 1 according to the invention. The physiological parameter measuring system 1 comprises a first and second contact lens 3a, 3b according to the invention and a first and second antenna patch 5a, 5b adhered to the glasses 7a, 7b of a pair of spectacles 9, on the side of the of the glasses towards a patient's eye. The antenna patches 5a, 5b are connected to a first and second electronic conditioning unit 11 a, 11 b, which are connected to each other. Furthermore, one of the electronic conditioning units, in this embodiment 11a, is configured to communicate with a data monitoring unit 13, e.g. using a wireless technology, Wifi, Bluetooth, Zigbee, ISM etc. The data monitoring unit 13 further comprises a data interface 15 via which the monitored data can be transmitted to an external device, for instance a computer 17, e.g. using a wired or wireless communication line.

According to a variant, the system 1 can only comprise one electronic condition unit being connected to both antenna patches 7a, 7b.

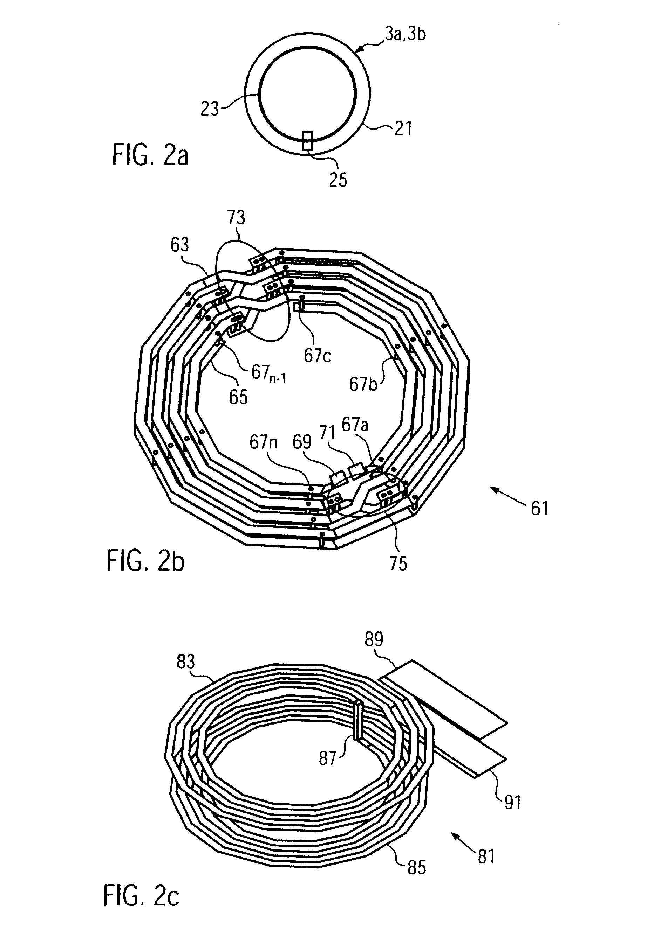

Figure 2a schematically illustrates the structure of each one of contact lenses 3a, 3b according to the invention. The contact lens comprises a contact lens base 21, comparable to a standard soft contact lens, which can have or not vision correction properties.

In addition, the contact lens 3a, 3b comprises an antenna 23 and a sensing means 25 which, are embedded into the contact lens base 12 to improve the wearing comfort. According to the invention, the sensing means 25 is a fully passive sensing means.

In this embodiment, the purely passive sensing means 25 is a capacitor, e.g. an off the shelf capacitor added to the antenna 23, or an integrated capacitor fabricated at the same time as the antenna loop, or a piezoelectric transducer transforming a mechanical excitation into an electric signal.

Antenna 23 and sensing means 25 effectively build up a passive device corresponding to a LC (inductance + capacitance) circuit. The physiological parameter measurement can then be based on the observation of a frequency drift of the resonance frequency as a function of cornea deformation. The inductance and capacitance are chosen such that a resonance frequency is observed in a frequency range allowed by the national safety regulations, in particular in the so called ISM band 26,957 to 27,283 MHz. To adapt the resonance frequency to such a frequency band, it is possible to adapt the number of spires, e.g. in a rang of about 1 to 25 spires, more in particular 1 to 5 spires, of the antenna 23 and to adapt the capacitance by using an appropriate additional capacitor in addition to the self capacitance of the antenna loop.

The contact lenses 3a, 3b according to the invention are fabricated in a two step fabrication process. In a practical example according to the invention, first of all, the antenna and sensing means are fabricated. The antenna 23 according to the invention can be formed by a first metal layer, in particular a copper layer, and a second layer on top thereof, in particular out of a biocompatible material like gold, with a spiral form. The first and second layer are provided on one main surface of a carrier substrate, e.g. a flexible material, like for instance polyimide which can be part of the capacitance of the LC circuit. To improve the sensitivity without having to enlarge the surface covered by the antenna a third layer of copper and a fourth layer of gold (to ensure biocompatibility) can be provided on the opposing other main surface of the carrier substrate, One pair of layers has a thickness in a range of about 20 to 40 µm whereas the flexible carrier substrate typically has a thickness in the range of 2 to 30µm, in particular 3 to 7 µm. If the carrier substrate is used as dielectric of the capacitance, a smaller thickness shall be chosen as the smaller the dielectric thickness, the higher capacitance integration density. A specific dielectric layer can also be deposited, creating an even lower capacitor as the capacitance integration is improved. The antenna 23 is connected to the capacitor (off the shelf or fabricated at the same time than the antenna) or piezoelectric transducer of the sensing means 25 before being mold in a biocompatible material, e.g. a PDMS (polydimethysiloxane) silicon polymer, serving as contact lens. According to a variant the flexible carrier with antenna 23 and capacitor can also be attached to a contact lens.

Instead of layer pairs (first and second on the one hand, and third and fourth on the other hand), the invention can also be realized with only one layer, or one layer on each side of the flexible carrier substrate. In this case the thickness is typically in the range of 10µm.

Figure 2b illustrates a first embodiment 61 of the LC circuit comprising the antenna and the sensing means according to the invention which at a later stage will become part of the contact lens 3a, 3b. In this first embodiment spires 63 and 65 are arranged on the front and backside of a flexible carrier substrate, which is not illustrated in this figure. Electric conductive vias, in particular metallic vias, 67a, 67b, ..., 67n-1, 67n connect the spires 63 on the one side and the spires 65 on the other side along their entire length. This arrangement has the advantage that the effective thickness of the spires is essentially doubled and thus the resistance can be reduced and the quality factor of the circuit enhanced. The design is chosen such that the two connecting parts 69 and 71 are side by side on one surface of the flexible carrier substrate to allow the connection to an additional capacitor. The design of figure 2a has two crossing areas 73 and 75 connecting an essentially semi circularly shaped part on an inner area with an essentially semi circularly shaped part in the subsequent round of the spires so that in the end the spires on one side of the substrate never cross more than one spires on the other side of the carrier substrate.

Figure 2c illustrates a second embodiment 81. Again spires 83 and 85 are arranged on the front and back side of the flexible carrier substrate, not illustrated. The spires are connected to each other with a via 87 going through the carrier substrate. The via connects the end of the first inductance with the beginning of the second inductance and both are thus forming two inductances connected in series. Both inductances are wound in the same direction, so that the total inductance is enhanced, without needing more space. Figure 2c furthermore illustrates two electrodes 89 and 91 connected to the beginning of the first inductance 83 and the end of the second inductance 85 thereby forming an integrated capacitance to adapt the resonant frequency to the desired frequency range.

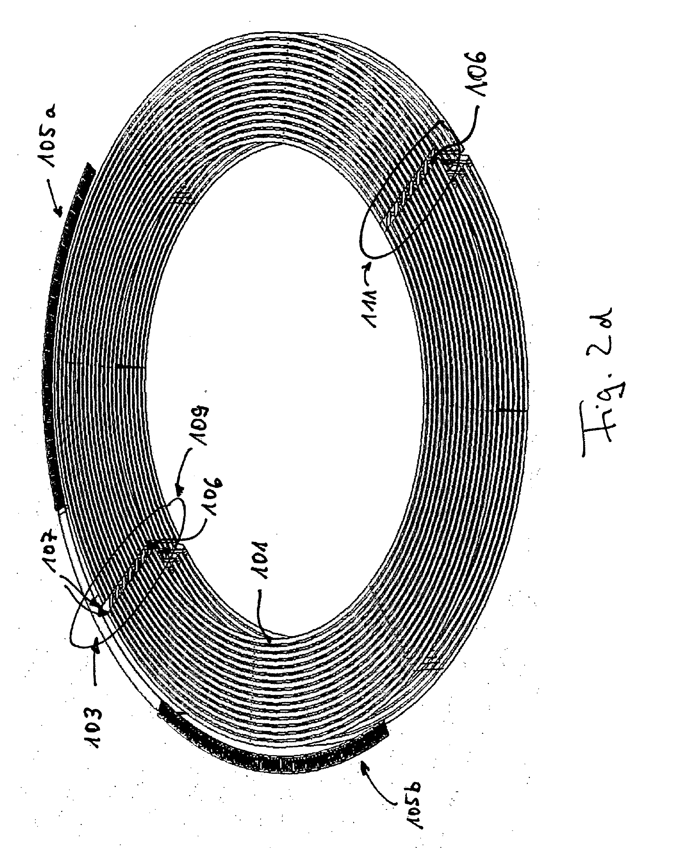

Figure 2d illustrates a third embodiment of the LC circuit of a contact lens according to the invention. The structure is similar to the one of the first embodiment, however, it comprises a larger amount of spires 101, namely 15, forming the inductance and an integrated capacitor 103. In addition, the LC circuit further comprises an interdigitated capacitance, here in fact two 105a and 105b, connected to the integrated capacitor 103. The interdigitated capacitance 105a, 105b serves to fine tune the resonance frequency.

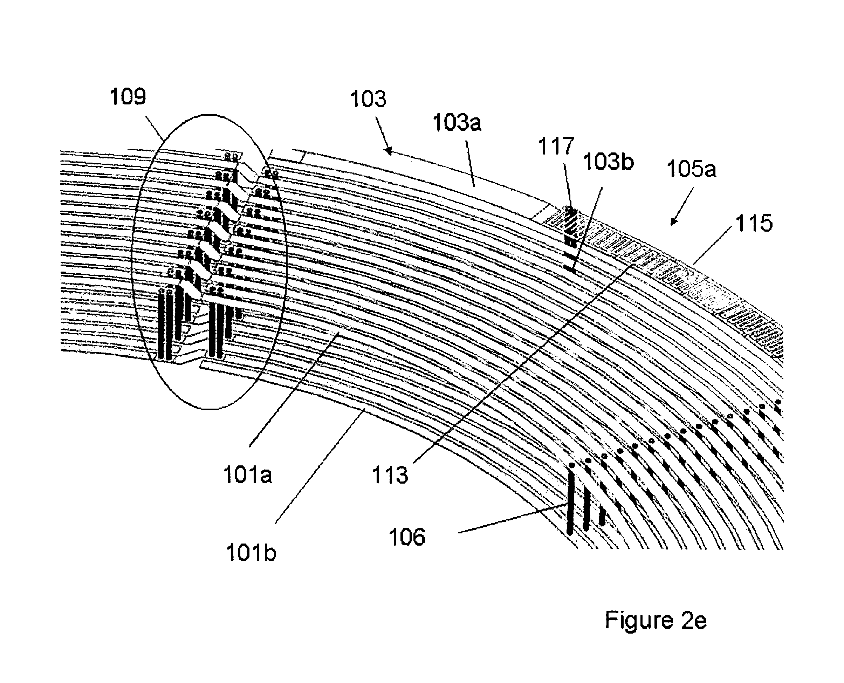

In this embodiment the upper and lower spires 101 (reference numerals 101 a and 101b in Figure 2e) are arranged on the front and backside of a flexible carrier substrate, which is again not illustrated in this figure. Electric conductive vias, in particular metallic vias 106, which are like in the first embodiment, connect the spires on the one side and the spires on the other side along their entire length. This arrangement has again the advantage that the effective thickness of the spires is essentially doubled and thus the resistance can be reduced and the quality factor of the circuit enhanced.

The design in the third embodiment is chosen such that the connection 107 towards the integrated capacitor 103 is outwardly with respect to the center of the spires. The design of figure 2d has, like the design of figure 2b, a plurality of crossing areas 109 and 111 connecting an essentially semi circularly shaped part on an inner area with an essentially semi circularly shaped part in the subsequent round of the spires so that in the end the spires on one side of the substrate never cross more than one spires on the other side of the carrier substrate.

The inductance value needs to be as large as possible to reduce the capacitor value and as a consequence the area required for its integration, large capacitor areas could actually alter the patient's comfort. To increase the inductor value, the number of spires 101 should be higher as in the first embodiment not having the integrated capacitance. With the design of the third embodiment it is possible to integrate about 10 to 20, here 15, spires without having to fill the centre region, which would again reduce the patient's comfort. With 10 to 20 spires on the surface area roughly corresponding to a contact lens, it is possible to achieve inductance values in the range 1500 to 5000nH. The area for the spires being also limited, a limitation on the spires width also exists. It is typically in a range of 70 to 100um. The consequence is an inductance resistivity that can be large, knowing the metal thickness is in general limited to about 10µm per layer, because of the deposition process and the need for a flexible structure to improve the sensor sensitivity which is also dependent on the structure thickness.

To lower the overall inductance resistivity and increase the quality coefficient of the overall LC structure, benefit is thus taken from the fact that at least two metal layers (one on each side of the substrate) are provided. The thickness of the spires 101 is essentially doubled by using the at least two metal layers which are connected together by means of the vias 106.

By achieving a "effective" higher metal thickness for the spires 101 provides the further advantages that the self parasitic capacitance is higher compared to a situation with only one layer, thereby decreasing the capacitance value of the integrated capacitance 103. Thus the integration area can be kept small which improves the patient's comfort. Further, the sensor sensitivity is increased since the sensitivity is driven by the self parasitic capacitance of the inductance. Finally, the design is arranged such that the self parasitic capacitance is such that the self resonance frequency (SRF) of the inductance remains always higher than the sensor's working frequency.

The capacitor 103 is build up by two metal plates (Metal-Insulator-Metal or MIM capacitor) with one metal plate on each side of the carrier substrate. Instead of using the carrier substrate, any dielectric could be placed between the two metal plates, e.g. Teflon or SiO2 depending on the deposition process used. The Metal-Insulator-Metal capacitor is used to tune the resonance frequency.

In this embodiment, the capacitor has an arc shaped form so that it adapts to the shape of the contact lens. Furthermore, in this embodiment the capacitance 103 is arranged on the outer side of the spires 101, seen from the center of the spires. Instead it could of course have any other suitable shape, e.g. circular or rectangular. As illustrated in figure 2d, the capacitor preferably does not for a complete ring around the spires 101 of the inductance because of a negative effect on the inductance value, which would be lowered.

With the capacitor 103 as illustrated in Figure 2d, a capacitance value of 0,5 to 20pF can be achieved.

The dielectric loss characteristic (also called tanδ) is important since this parameter drive the ESR (Equivalent Series Resistor) characteristics of the MIM capacitor. A high ESR value will offer poor performances for the LC circuit quality factor. The ESR parameter is as important as the resistivity of the inductor since both the inductor and capacitor are electrically connected in series.

The inter-digitated capacitances 105a and105b are used to fine tune the resonance frequency of the sensor during electrical tests operations such that it stays within the desired frequency band. As illustrated in figure 2e showing an enlarged detail of figure 2d, an inter-digitated capacitance 105a is realized with interlaced metal fingers. Fingers 113 are connected to the upper metal layer 103a of the capacitor 103 and the fingers 115 are connected via vias 117 with the lower metal layer 103b of the capacitor 103. During calibration of the LC circuit it is possible to reduce the capacitance value of the circuit and thus increase and tune the resonance frequency by cutting or a metal ablation operation on one or more fingers with a laser beam. For instance, the embedded inter-digitated capacitance 105a, 105b has a value in the range of 0,6pF for an operation in the 27MHz ISM band.

In this embodiment, the shape of the inter-digitated capacitance 105a, 105b is arc shaped with the same radius as the capacitor 103 and the inter-digitaed capacitances 105a, 105b are connected at both extremities of the capacitor 103 and are arranged on one side of the carrier substrate. According to variants only one inter-digitated capacitance 105a could be provided and or additional inter-digitated capacitances could be provided on both sides of the carrier substrate.

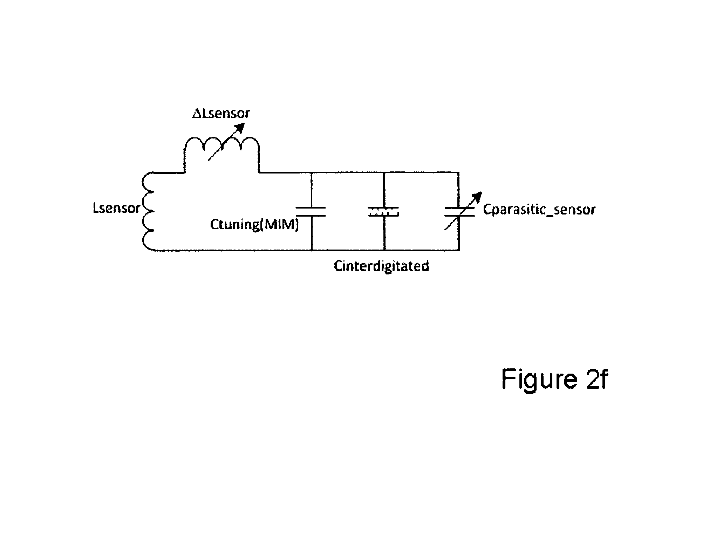

Figure 2f illustrates the electrical equivalent circuit of the contact lens 3a, 3b according to the third embodiment of the invention. The spires 101 form the sensing inductance Lₛₑₙₛₒᵣ also representing a varying part ΔLₛₑₙₛₒᵣ The pressure dependant capacitance changing as a function of contact lens deformation as a consequence of the cornea deformation corresponds to Cₛₑₙₛₒᵣ and the additional capacitance 103 to tune the resonant frequency to the desired value corresponds to Cₜᵤₙₑ. Finally, the inter-digiated capacitance 105a, 105b, used to fine tune during calibration is indicated as C_{interdigitated} The resistance of the overall circuit design is not represented.

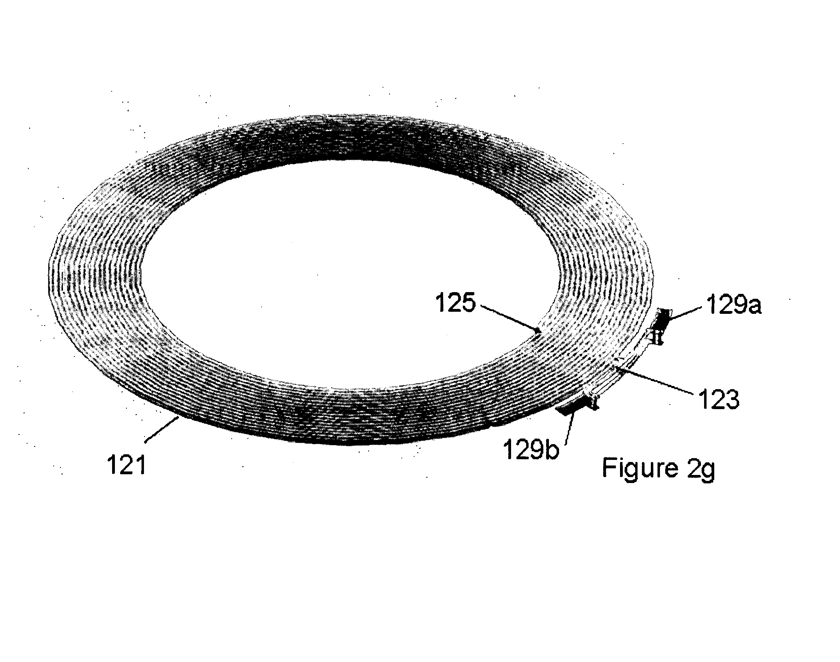

Figure 2g illustrates a fourth embodiment of the inventive contact lens. Spires 121 are arranged on the front side of the flexible carrier substrate, not illustrated. The inner end of the spires 121 is connected via a via 125 going through the carrier substrate with a connecting element 123 connecting the inner end of the spires 121 with the lower electrode of a capacitor 127. Figure 2g illustrates the capacitor 127 as an arc-shaped integrated capacitor 127 build-up like in the third embodiment towards the outside of the spires 121 forming an integrated capacitance to adapt the resonant frequency to the desired frequency range. The upper electrode of the capacitor is connected to the beginning of the first inductance 121. Finally, in the same way as in the third embodiment, two inter-digitated capacitances 129a and 129b are connected to the integrated capacitor 127.

According to the variant, the linear connecting element 123 could be replaced by a second set of spires like in the second embodiment illustrated in Figure 2c, thus forming a series of the inductances. The second set of spires would have to be wound in the same direction.

Of course, the integrated capacitance could also be provided in the first embodiment, just like the design of any one of the second to fourth embodiment could be connected to an external capacitance. In general any feature described above for at least one of the embodiments can also be incorporated into any one of the remaining embodiments to achieve further inventive embodiments of the invention.

With the all passive design, it is possible to embed thin components, like already mentioned in the order of 20 to 40µm per layer pair, in the contact lens, the thickness of which can be kept in the order of 100µm which greatly enhances the wearing comfort compared to the prior art device which due to the presence of the active strain gauge, is thicker, typically at least 150µm corresponding to a thickness at which the wearing comfort for the patient reduces rapidly. In addition, the thinner the lens can be made, the more sensitive the IOP measurement. Indeed, a change in pressure can be better indentified for a thin lens than a thick lens which is more rigid.

The use of a purely passive sensing means has the advantage that, compared to active sensing means, the power consumption in the circuit of the contact lens is greatly reduced, so that radiation and energy dissipating phenomena, which may harm the patient's eye, can be kept at a lower level compared to the prior art using active strain gauges.

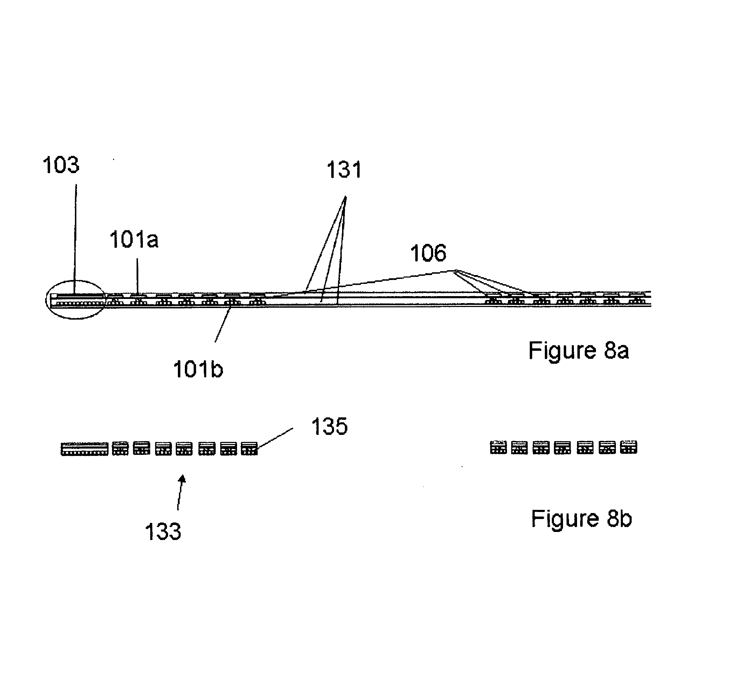

Figures 8a to 8d illustrate a fabrication process according to a fifth embodiment of the invention to integrate a LC circuit of a sensor like illustrated in Figure 2d with a contact lens, in particular a soft contact lens. Instead of the third embodiment, also a LC Circuit according to one of the other embodiments could be integrated with a soft contact lens in the same way.

Once the electrical tests and fine tuning operations have been carried out on an LC circuit of a sensor to measure IOP, the sensor is prepared for its integration with the soft contact lens.

Figure 8a illustrates the LC circuit of Figure 2d in a cross cut view. The cut view illustrates the capacitor 103 the top and bottom spires 101 a and 101 b and the vias 106 connecting the spires 101 a and 101 b. In this cut view, the inter-digitated capacitor 105a, 105b cannot be seen. Figure 8a furthermore illustrates the flexible carrier substrate 131 already described above. In this embodiment, the flexible carrier substrate 131 is a polyimide layer, but other materials could also be used.

According to a variant, the carrier substrate could also be rigid since, the spires are placed on top of it and due to the material removal carried out in the next step (Figure 8b), the shape can still be adapted to the one of a contact lens. This is even more true if the spire width is very small.

As a next step, illustrated in Figure 8b, the substrate material between the spires is removed. This step is for instance achieved, with a selective laser ablation. Like illustrated in Figure 8b, the sensor structure 133 comprises carrier substrate material 135 between the upper and lower spires 101a and 101b. In the center region no material remains which improves the patient's comfort.

Eventually a thin layer of the carrier substrate remains on the side of the upper spires 101 a. In this case the laser ablation process is carried out from the backside of the carrier substrate, thus the one with the lower spires 101 b.

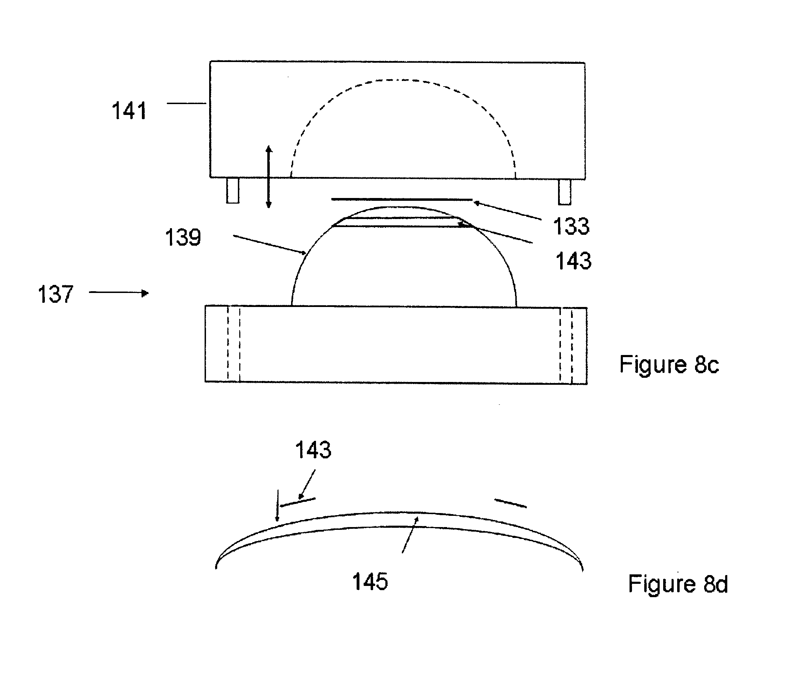

Next, as illustrated in Figure 8c, a curved shape is given to the sensor structure 133 so that it fits to the shape of a contact lens, e.g. a soft contact lens. To do so the sensor structure 133 is positioned within a forming tool 137 comprising a male part 139 and a female part 1341. The sensor structure is positioned on the male part 139 comprising a shape with a standard lens radius. By closing the forming tool 137, guiding protrusions on the female part 141 enter into mating guide holes in the male part 139 to ensure a proper alignment, the formerly flat sensor structure 133 is deformed into a form 143 following a contact lens' shape.

Due to the fact that the curved shape introduces a resonance frequency offset compared to flat shape, the resonance frequency tuning with the inter-digitated capacitor is achieved considering this offset. Next the bent sensor structure 143 is then attached to a contact lens, e.g. an off the shelf soft contact lens with or without view correcting properties.

Eventually the bent sensor structure 143 or the unbent structure 133 is beforehand embedded into a material that is compatible with the contact lens material. As can be seen in Figure 8d, the bent structure is provided on the side of the contact lens 145 that is away from a persons eyeball. Thus, on the eyeball the patient will not feel any difference to a standard contact lens and due to the thin final structure the overall wearing comfort can be kept high.

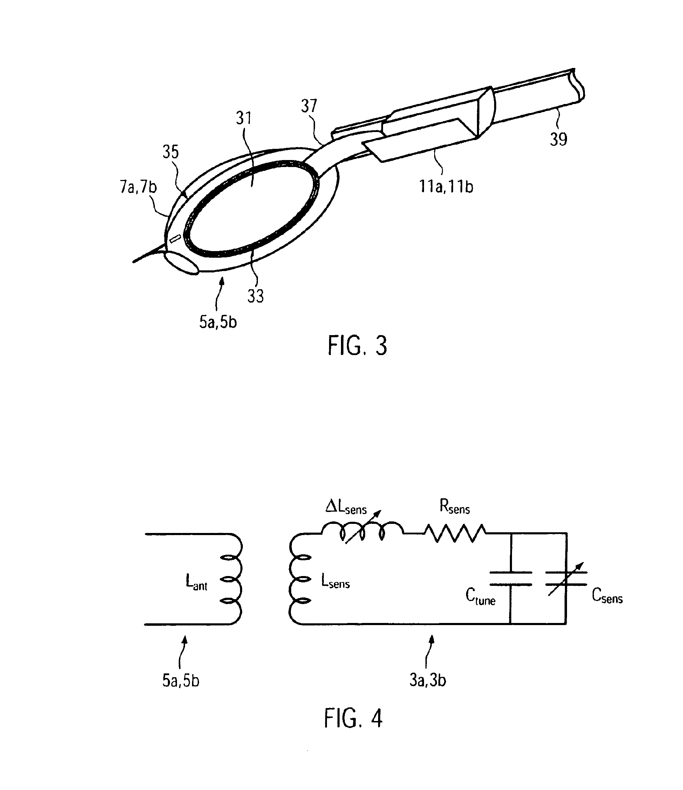

Figure 3 illustrates a partial 3-dimensional view of one of the antenna patches 5a, 5b and the corresponding conditioner 11 a, 11 b.

According to a sixth embodiment of the invention, the antenna patch 5a, 5b comprises a transparent support layer 31, an antenna loop 33 which, according to a variant, could also be transparent, as well as an adhesive layer 35 used to attach each antenna patch 5a, 5b to one of the glasses 7a, 7b of the spectacles respectively. The adhesive 35 is chosen such that the antenna patch can be removed from the glass 7a, 7b without leaving a trace or without deteriorating the glass 7a, 7b of the spectacles.

This antenna patch 5a, 5b can thus be attached to any glasses with or without vision correction. For instance, the glasses of the spectacles of the patient which he uses in his daily life can be used in the monitoring system 1.

Preferably antenna patches 5a, 5b with various shapes can be provided to be compatible with any form and design of spectacle glasses.

An electric connector 37 links the antenna patch to an electronic conditioning unit 11a, 11b being attached or clamped to the branches 39 of the spectacles. In this embodiment, the electrical connection 37 is pinched to the electronic conditioning unit 11 a, 11 b.

Figure 4 illustrates the electrical equivalent circuit of the contact lens 3a, 3b and the antenna patch 5a, 5b. The antenna loop 33 corresponds to the antenna inductance Lₐₙₜ, the antenna 23 to the sensing inductance Lₛₑₙₛ also representing a varying part ΔLₛₑₙₛ, the pressure dependant capacitance changing as a function of contact lens deformation as a consequence of the cornea deformation corresponds to Cₛₑₙₛ, and the eventually present additional capacitance to tune the resonant frequency to the desired value corresponds to Cₜᵤₙₑ. The resistance of the overall circuit design is represented by Rₛₑₙₛ.

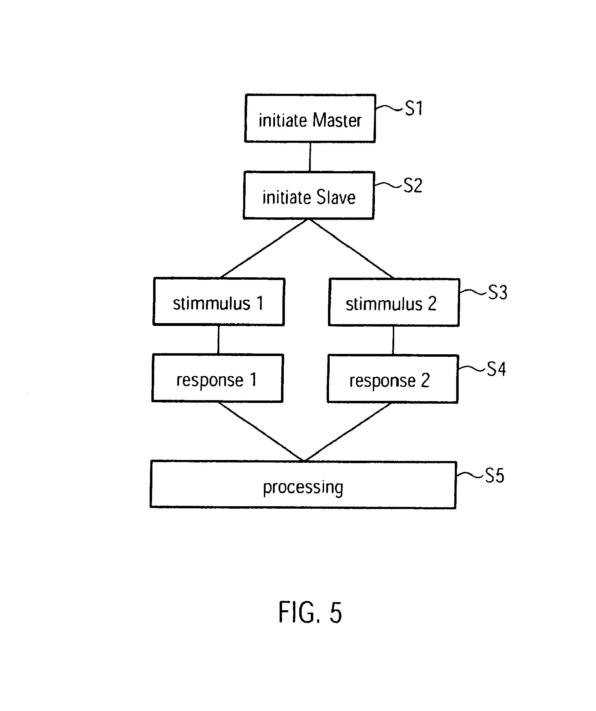

The inventive physiological parameter measuring system as illustrated in Figures 1 - 3 is used according to the following inventive method according to a seventh embodiment to measure a physiological parameter, in particular the intraocular eye pressure. The method is illustrated in Figure 5.

The electronic conditioning unit 11 a, serving as the master electronic conditioning unit, transmits a signal to initiate the slave electronic conditioning unit (Step S2). Subsequently stimulus signals are transmitted via the antennas of the antenna patches 5a, 5b inductively coupled to the antennas 23 of the contact lenses 3a, 3b (Step S3). The contact lenses 3a, 3b then in turn transmit their response signals from the sensing means 25 in each one of the contact lenses 3a, 3b back to the antenna patch (Step S4) so that the response signal can be received by the electronic conditioning unit 11 a, 11 b for further processing (Step S5), either within one or both of the electronic conditioning units 11a, 11 b or the data monitoring unit 13. The signals in the data monitoring unit 13 are treated in a programmable integrated circuit such as a DSP (Digital Signal Processor), or an FPGA (Field Programmable Gate Array) so that the system 1 is fully programmable with respect to the type of stimulus, the type of pre and post-processing, and thus adaptable to any special demand.

The physiological parameter measurement and here in particular the IOP measurement with the system 1 according to the invention is based on the following method. By combining the antenna patch's excitation voltage with the current going though the antenna loop 33, it is possible to compute the mathematically complex impedance seen from the antenna. A complex impedance Z (with Z = α+jβ) is characterized by a modulus (M) and a phase (Φ). The resonance frequency of the LC circuit of the antenna 23 and the sensing means 25 shifts with IOP variations and the impedance phase (Φ(Z)) goes through a minima at the resonance frequency of the LC circuit on the lens 3a, 3b. Doing a frequency sweep on the antenna patch 5a, 5b thus permits to find out the phase minima and thus the frequency offset due to the IOP variations. The frequency shifts information are transmitted to the data monitoring unit 13 which, after validation of the measurement, is computed into IOP variations by taking into account cornea thickness information, measured on the patient just before this one is equipped with device. The invention thus gives access to the pressure variations which represent an important aspect for glaucoma patients.

The use of the frequency based measurement has the advantage that it is non-sensitive to the amplitude of the signal and consequently also independent of the distance between the glasses of the spectacles and the contact lens 3a. Nevertheless using the transparent antenna patches 5a, 5b, the antenna loop 33 can be placed right in front of the contact lens 3a and 3b. Thus the coupling between the two circuits can be optimized while at the same time wearing comfort can be kept high.

In the embodiments as illustrated in Figures 1 - 3, two contact lenses, two antenna patches and two conditioning units are used, however, without departing from the inventive idea of using only a passive device in the contact lens, the physiological parameter measuring system could be further simplified by only using one contact lens, one antenna patch and one conditioning unit.

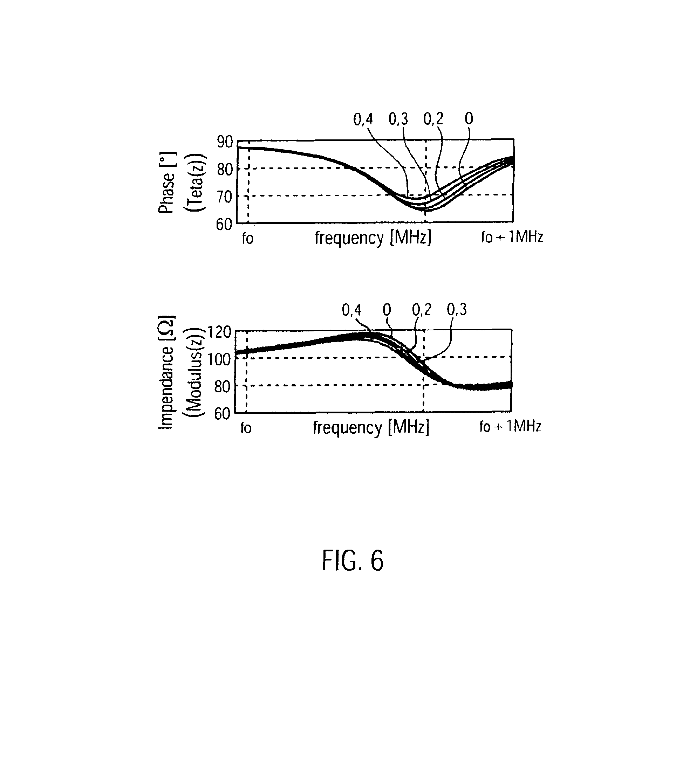

Figure 6 illustrates the results of a frequency and phase measurement using a contact lens with an antenna 23 like illustrated in Figure 2b. To obtain a resonant frequency in the desired range an additional capacitance was added, here an MLCC (Multi-Layer Ceramic Capacitor) type capacitor.

The measurement results illustrated in Figure 6 were obtained by placing the contact lens 3a on an enucleated pork eye with the antenna patch 5a positioned 1,5 cm above the lens. The pressure in the eye was varied by injecting a fluid. In this practical example the four curves correspond to 0ml air injected (about 20mmHg IOP), 0,2ml air injected (about 70mmHg IOP), 0,3 ml air injected (about 85mmHg IOP) and 0,4 ml air injected (about 90mmHg IOP). Whereas the upper figure illustrates the change in the impedance, the lower figure illustrates the change in phase for IOP pressure changes in the order of 70mmHg a frequency shift of about 77KHz was observed.

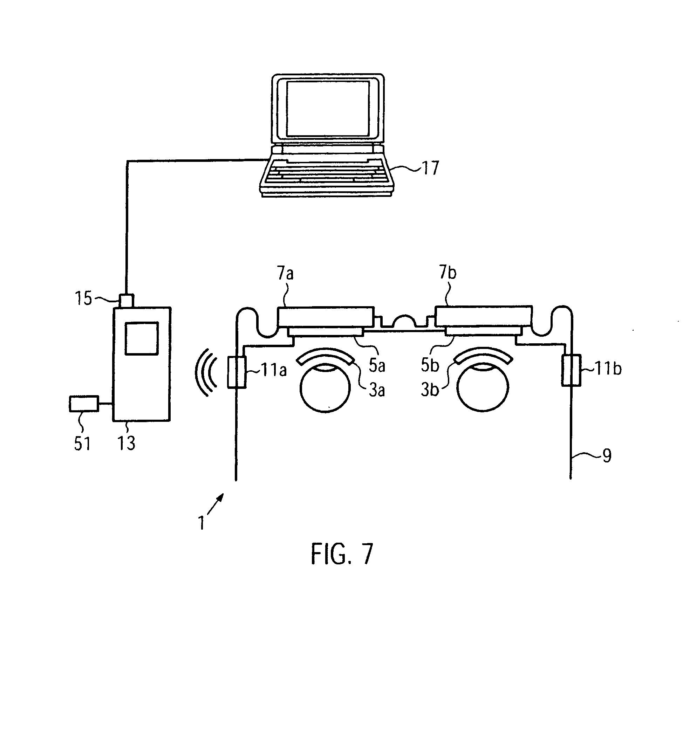

Figure 7 illustrates a eighth embodiment according to the invention, wherein the physiological parameter measuring system 41 comprises, in addition, a filtering unit 43 which is configured to remove spurious artifacts which are observed in the measured signals and which can be due to eye blinking or a change of the viewing direction which lead to predetermined changes in the signals which can be removed by the filtering unit 43 to improve accuracy of the system. As eye blinking and change of viewing direction is typically a synchronized event between the left and the right eye, the observation of the predetermined change in the signal simultaneously in both signal channels is used to trigger a filtering event. To improve the signal-to-noise ration, electromagnetic disturbances of the signals can also be eliminated in the filtering unit based on the dual-channel measurement, as they are present in both response signals when occurring. In this embodiment, the filtering is based on digital signal processing. The filtering unit 43 of this embodiment is embedded in the data monitoring unit 13. As mentioned above, it is easy to detect a simultaneous event on both channels (e.g., same shape in the spectrum).

## Claims

1. Sensing means for a contact lens, with an inductance comprising first spires (101 a) on a first main side of a carrier substrate and second spires (101 b) on the second main surface, opposite the first surface, of the carrier substrate and a capacitance.

2. Sensing means for a contact lens according to claim 1, wherein the capacitance (103) is integrally formed with the inductance, wherein the one electrode is on the first main side and the second electrode is on the second main side of the carrier substrate and the dielectric of the capacitance is a part of the carrier substrate.

3. Sensing means for a contact lens according to claim 2, wherein the capacitance is arranged towards the outside of the spires when looking from the centre of the spires.

4. Sensing means for a contact lens according to claim 2 or 3, wherein the capacitance is arc-shaped and extends over less than an entire circle.

5. Sensing means for a contact lens comprising an inductance and a capacitance, in particular according to one of claims 1 to 4, further comprising at least one interdigitated capacitance (115a, 115b), in particular connected to the capacitance (103).

6. Sensing means according to claim 5, wherein the interdigitated capacitance is arc-shaped.

7. Sensing means according to one of claims 1 to 6, wherein the two connecting parts (69, 71) of the spires are both either arranged towards the inside of the spires or the outside of the spires.

8. Sensing means according to claim 7, wherein the connecting parts (69, 71) are arranged on the same side of the flexible carrier substrate.

9. Sensing means according to one of claims 1 to 7, wherein the first spires (63, 101 a) and the second spires (65, 101 b) are connected via a plurality of vias (67ₙ, 106) over the entire length of the spires.

10. Sensing means according to claim 9, wherein the sensing means comprises two crossing areas (73 and 75, 109 and 111) where an essentially semi circularly shaped part of the spires connects with another essentially semi circularly shaped part in the subsequent round of the spires.

11. Sensing means according to claim 10, wherein the connection between two essentially semi circularly shaped parts of the spires is either achieved on the first or the second main surface side.

12. Sensing means according to claim 10 or 11, wherein the connected essentially semi circularly shaped part of the spires extend from the outside towards the inside and then back from the inside to the outside.

13. Sensing means according to claim 12, wherein each spire going inward crosses only one spire going outward and vice versa.

14. Sensing means according to one of claims 1 to 8, wherein the first spires and the second spires are connected via one via (125) at an end of the first and the beginning of the second spires wherein the spires are wound in the same direction to form a series of two inductances.

15. Sensing means according to one of claims 1 to 14, wherein the carrier substrate is at least partially removed in areas without spires (101 a, 101 b) and/or the capacitor (103) and/or the interdigitated capacitor (105a, 105b).

16. Contact lens for a physiological parameter measuring system comprising one or two contact lenses, comprising a sensing means according to one of claims 1 to 15.

17. Method for fabrication a contact lens according to claim 16 comprising the steps of:
a) Providing a sensing means according to one of claims 1 to 15,
b) Removing the carrier substrate at least partially in areas between spires and/or the capacitor and/or the interdigitated capacitor,
c) Plastically deforming the sensing means to obtain a bended shape corresponding to the shape of a contact lens, and
d) Attaching the bended sensing means to the contact lens.

18. Method for measuring a physiological parameter, in particular the intra ocular eye pressure, comprising the steps of:
a) transmitting a stimulus signal from an antenna patch, in particular adhered to the glasses of spectacles to a purely passive sensing means of a contact lens,
b) receiving a response signal from the purely passive sensing means via the antenna patch, and
c) determining the physiological parameter based on a resonant frequency and/or phase shift.
